(19) Europäisches Patentamt
European Patent Office
Office européen des brevets

(11) Publication number : **0 480 693 A2**

# (12) EUROPEAN PATENT APPLICATION

(21) Application number : **91309252.4**

(22) Date of filing : **09.10.91**

(51) Int. Cl.$^5$ : **C07H 19/01,** A01N 43/90, A61K 31/70, C07D 493/22

(30) Priority : **11.10.90 US 595872**

(43) Date of publication of application : **15.04.92 Bulletin 92/16**

(84) Designated Contracting States : **CH DE FR GB IT LI NL**

(71) Applicant : **MERCK & CO. INC.**
**126, East Lincoln Avenue P.O. Box 2000**
**Rahway New Jersey 07065-0900 (US)**

(72) Inventor : **Shih, Thomas L.**
**16 Jersey Avenue**
**Edison, NJ 08820 (US)**

(74) Representative : **Thompson, John Dr. et al**
**Merck & Co., Inc. European Patent**
**Department Terlings Park Eastwick Road**
**Harlow, Essex CM20 2QR (GB)**

## (54) Avermectin degradation products and derivatives.

(57) Novel avermectin derivatives are disclosed, wherein the C-17-21-25-dioxaspirane substructure has been disassembled and modified to include oxygen substitution at C-24 and C-25 and examples of C-24 and C-25 substituents are hydrogen, alkyl, alkenyl, substituted alkyl or substituted alkenyl groups. These compounds can be further substituted at the 4″-, 5-, 13-, and 23- positions. The new C-24 and C-25 substituted avermectin derivatives are prepared by cleavage of known and suitably protected avermectin compounds. The new compounds are potent anti-parasitic agents, in particular, the compounds are anthelmintic, insecticidal and acaricidal agents.

EP 0 480 693 A2

BACKGROUND OF THE INVENTION

The term avermectin (previously referred to as C-076) is used to describe a series of compounds isolated from the fermentation broth of an avermectin producing strain of Streptomyces avermitilis and derivatives thereof. The morphological characteristics of the culture are completely described in U.S. Patent No. 4,310,519. The avermectin compounds are a series of macrolides, each of which is substituted at the 13 position with a 4-(alpha-L-oleandrosyl)-alpha-L-oleandrose group. The avermectin compounds and the instant derivatives thereof have a very high degree of anthelmintic and anti-parasitic activity.

The avermectin series of compounds isolated from the fermentation broth have the following structure:

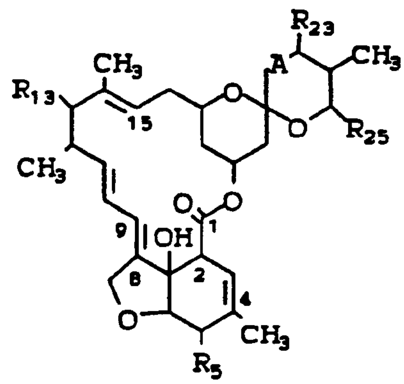

wherein $R_{13}$ is the 4'-(α-L-oleandrosyl)-α-L-oleandrosyloxy group of the structure:

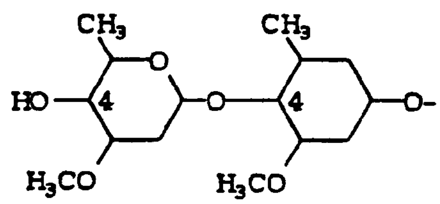

and wherein A at the 22,23 position indicates a single or a double bond; $R_{23}$ is a hydrogen or hydroxy and is present only when A indicates a single bond;

$R_{25}$ is iso-propyl or sec-butyl; and

$R_5$ is methoxy or hydroxy.

There are eight different avermectin natural product compounds and they are given the designations A1a, A1b, A2a, A2b, B1a, B1b, B2a, and B2b based upon the structure of the individual compounds.

In the foregoing structural formula, the individual avermectin compounds are as set forth below. (The $R_{13}$ group is 4'-(α-L-oleandrosyl)-α-L-oleandrosyloxy:

| | (A) | $R_{23}$ | $R_{25}$ | $R_5$ |
|---|---|---|---|---|
| A1a | double bond | -- | sec-butyl | $-OCH_3$ |
| A1b | double bond | -- | iso-propyl | $-OCH_3$ |
| A2a | single bond | -OH | sec-butyl | $-OCH_3$ |
| A2b | single bond | -OH | iso-propyl | $-OCH_3$ |
| B1a | double bond | -- | sec-butyl | -OH |
| B1b | double bond | -- | iso-propyl | -OH |
| B2a | single bond | -OH | sec-butyl | -OH |
| B2b | single bond | -OH | iso-propyl | -OH |

The avermectin compounds are generaly isolated as mixtures of a and b components. Such compounds

differ only in the nature of the $R_{25}$ substituent and the minor structural differences have been found to have very little effect on the isolation procedures, chemical reactivity and biological activity of such compounds.

In addition to these natural avermectins containing the 25-iso-propyl or 25-sec-butyl- substituent, closely related derivatives containing other branched or cyclic 25-alkyl or 25-alkenyl substituents, optionally further substituted by heteroatoms such as oxygen, sulfur, nitrogen, and halogen, are known in the literature. These derivatives are obtained through various adjustments and additions to the fermentation procedures as described fully in the European Patent Application EPO 0 214 731.

Avermectins are products of microbial fermentations using the actinomycete Streptomyces avermitilis. These microbes use acetates and propionates as building blocks for most of the avermectin carbon chain, which is then further modified by microbial enzymes to give the completed avermectin molecules. It is known, however, that the carbon C-25 and the 2-propyl and 2-butyl substituents at this carbon are not derived from acetate or propionate units, but are derived from aminoacids L-valine and L-isoleucine, respectively. It was reasoned, that these aminoacids are deaminated to the corresponding 2-ketoacids, and that these then are decarboxylated to give 2-methylpropionic and 2-methylbutyric acids. These acids then have been found to be directly incorporated into the avermectin structures to give the 2-propyl and 2-butyl C-25 substituents, as is reported by Chen et al., Abstr. Pap. Am. Chem. Soc. (186 Meet.,MBTD 28, 1983).

It was also disclosed in European Patent Application number 0 214 731 that additions of large amounts of other acids such as cyclopentanoic, cyclobutyric, 2-methylpentanoic, 2-methylhexanoic, thiophene-3-carboxylic acids and others to the fermentation broth of S. avermitilis causes the microbes to accept these acids as substitutents and to make small amounts of avermectins containing these acids in form of new C-25 substituents. Examples of such new avermectin derivatives are:

25-(thien-3-yl)-25-de-(1-methylpropyl)avermectin A2a
25-(cyclohex-3-enyl)-25-de-(1-methylpropyl)avermectin A2a
25-cyclohexyl-25-de-(1-methylpropyl)avermectin A2a
25-(1-methylthioethyl)-25-de-(1-methylpropyl)-avermectin A2a
25-(2-methylcyclopropyl)-25-de-(1-methylpropyl)-avermectin A2a

Similar experiments producing avermectins "c" and "d" containing as C-25 substituents a 2-pentyl and 2-hexyl group are described by T. S. Chen et al. in Arch. Biochem. Biophys. 1989, 269, 544-547.

Still additional avermectin derivatives are produced through artificial modification of the fermentation of Streptomyces avermitilis either by addition of metabolic inhibitors such as sinefungin (as described by Schulman et al., J. Antibiot. 1985, 38, 1494-1498) or by mutation of the parent strain (as described by Schulman et al., Antimicrobial Agents and Chemotherapy, 1987, 31, 744-747, and by EP-276-131-A to Pfizer Inc.). Some of these avermectin derivatives are still further modified and are missing one or two of the 3′- and 3″-O-methyl groups (Schulman et al., J. Antibiot. 1985, 38, 1494-1498). Examples for such derivatives are:

3′,3″-Bisdesmethylavermectin B1a and B1b
3′,3″-Bisdesmethylavermectin B2a and B2b
3″-Desmethylavermectin B1a and B1b
3″-Desmethylnvermectin B2a and B2b
3′,3″-Bisdesmethyl-25-cyclohexyl-25-de-(2-butyl)-avermectin B2a
3′,3″-Bisdesmethyl-25-cyclopentyl-25-de-(2-butyl)-avermectin B2a
3′,3″-Bisdesmethyl-25-(3-thienyl)-25-de-(2-butyl)-avermectin B2a
3′,3″-Bisdesmethyl-25-(3-furyl)-25-de-(2-butyl)-avermectin B2a
3′,3″-Bisdesmethyl-25-(1-methylthioethyl)-25-de-(2-butyl)-avermectin B1a.

Milbemycin compounds are similar to the above avermectin compounds in that the 16-membered macrocyclic ring is present. However, such compounds are unsubstituted at the 13-position and have a methyl or ethyl group at the 25 position (the position the $R_{25}$ group as found in the above structure). Such milbemycin compounds and the fermentation conditions used to prepare them are described in U. S. Pat. No. 3,950,360. In addition, 13-deoxy-avermectin aglycones are prepared synthetically from the avermectin natural products and are disclosed in U. S. Pat. Nos. 4,171,134 and 4,173,571. Such compounds are very similar to the milbemycins differing from some of the milbemycins in having an isopropyl or sec-butyl rather than a methyl or ethyl group at the 25-position. Still other milbemycin type structures are natural products described in Eur. Pat. App. EPO 170,006 and U. K. Pat. App. 2,166,436 and are named LL-F28249 Antibiotic Complex or Antibiotics S541 or nemadectin. These are distinguished from the milbemycins and 13-deoxyavermectin aglycones by having a C-25 alkyl substituent containing an unsaturation.

The fermentation products have been chemically modified in order to obtain further antiparasitic and insecticidal analogs with improved properties such as potency, safety, antiparasitic spectrum, solubilities, stabilities and application forms. Publications of such procedures in the scientific and patent literature have been reviewed by Fisher, M. H.; Mrozik, H. In Macrolide Antibiotics; Omura, S., Ed.; Academic: New York, 1984; pp 553-606,

and by Davies, H. G.; Green, R. H. Nat. Prod. Rep., 1986, 3, 87-121.

For example, a group of semisynthetic avermectin derivatives were obtained by hydrogenating specifically the 22,23-double bond of avermectin B1 giving 22,23-dihydroavermectin B1 derivatives which have very potent anthelmintic and antiparasitic properties with a sufficient safety margin to allow mass application to humans infected with the filarial parasite causing the tropical disease onchocerciasis or "River Blindness". Another group of semisynthetic 10,11-dihydro avermectin B1 derivatives have longer persistent activities as agricultural miticides and insecticides due to increased photostability. Still other examples of semisynthetic avermectin derivatives contain a 8,9-oxide group, a 4a-hydroxy or acyloxy group, a 23-keto group, which all are potent antiparasitic and insecticidal compounds. It has also been described by Mrozik in United States Patent No. 4,427,663 that amino substituents at the 4"- and 4'- positions have very high antiparasitic and insecticidal activities.

These compounds may be used as starting materials for the compounds of the instant invention without further modification, or when containing additional reactive groups, which are not to be modified under the reaction conditions applied, only after protection of such with a suitable protecting group.

## SUMMARY OF THE INVENTION

The instant invention is concerned with derivatives of avermectin compounds wherein the C-24 and C-25 carbon atoms are substituted by hydrogen, oxygen, cyano, alkyl, alkenyl, substituted alkyl or substituted alkenyl groups. These compounds can be further substituted at the 4"-, 5-, 13-, and 23-positions. Thus, it is the object of this invention to describe such compounds. It is a further object of this invention to describe the processes useful for the preparation of such compounds. A still further object is to describe the use of such compounds as anthelmintic, insecticidal, and acaricidal agents. Still further objects will become apparent from the reading of the following description.

## DESCRIPTION OF THE INVENTION

The compounds of the instant invention have the following structural formula:

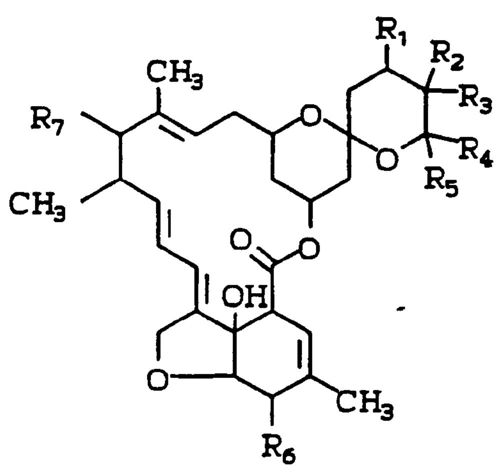

where

$R_1$ is hydrogen, hydroxy, oxo, halogen, =NOH or =$NOCH_3$;
$R_2$ is hydroxy, halogen or cyano; and
$R_3$ is methyl; or
$R_1$ and $R_2$ are combined to form a 23,24-epoxide; and
$R_3$ is methyl; or
$R_1$ and $R_2$ are combined to form a 23,24-double bond; and
$R_3$ is hydroxymethyl, loweralkoxyethyl, loweralkanoyloxymethyl, or halomethyl; and
$R_4$ is an alpha-branched loweralkyl or loweralkenyl group;
$R_5$ is hydrogen or hydroxy;
$R_5$ is hydroxy, loweralkoxy, oxo, =NOH, =$NOCH_3$, triloweralkyl substituted silyloxy, loweralkanoyloxy or phenoxy-loweralkanoyloxy;

$R_7$ is hydrogen, hydroxy, loweralkoxy, loweralkanoyloxy,

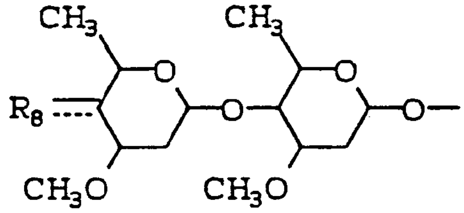

or

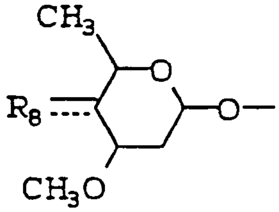

where $R_8$ is connected to the 4′ or 4″ carbon atoms by a single bond and is hydroxy, loweralkanoyloxy, loweralkoxy, amino, N-loweralkylamino, N,N-diloweralkylamino, N-loweralkyl-N-loweralkanoyl amino, N-loweralkyl substituted silyloxy or phenoxyloweralkanoyloxy; or $R_8$ is attached to the 4′ or 4″ carbon atoms by a double bond and is oxo, semicarbazono, N-loweralkylsemicarbazono, N,N-diloweralkylsemicarbazono, loweralkanoylhydrazono, benzoylhydrazono or loweralkylbenzoylhydrazono.

Preferred compounds of the instant invention are realized in the above structural formula when $R_1$ and $R_2$ together form a 23,24-double bond and $R_3$ is hydroxymethyl, loweralkoxymethyl, loweralkanoyloxymethyl or halomethyl.

Additional preferred compounds are realized when $R_1$ and $R_2$ together form a 23,24-epoxide and $R_3$ is methyl.

Still further preferred compounds are realized when $R_1$ is hydroxy, oxo, =NOH or =$NOCH_3$; $R_2$ is hydroxy, cyano or halogen; and $R_3$ is methyl.

Preferred compounds of the instant invention are further realized in the following compounds:

22-hydro-23,24-epoxyavermectin B1a/1b
24-hydroxyavermectin B2a/2b
25-hydroxyavermectin B2a/2b
22-hydro-Δ23,24-24a-hydroxyavermectin B1a/1b 4″-epiacetylamino-4″-deoxy-23,24-epoxyavermectin B1a/1b
4″-deoxy-4″-epiacetylamino-24-cyanoavermectin B2a/2b
4″-deoxy-4″-epiacetylamino-24-hydroxyavermectin B2a/2b
4″-deoxy-4″-epiacetylamino-25-hydroxyavermectin B2a/2b
4″-deoxy-4″-epiacetylamino-22-hydro-Δ23,24-avermectin B1a/1b
4″-deoxy-4″-epiacetylamino-22-hydro-Δ23,24-24a-hydroxyavermectin B1a/1b
22,23-dihydro-23-fluoroavermectin B1a/1b 4″-deoxy-4″-epiacetylamino-22,23-dihydro-23-fluoroavermectin B1a/1b
22-hydro-23-hydroxy-24-Cyanoavermectin B1a/B1b
25-Hydroxy-25-dehydro-avermectin B2a/B2b
24-Hydroxy-avermectin B2a/B2b.

In the instant invention the term "loweralkyl" is intended to indicate those alkyl groups of from 1 to 6 carbon atoms in a straight or branched chain configuration such as methyl, ethyl, propyl, isopropyl, butyl, pentyl, hexyl, and the like

The term "loweralkoxy" is intended to include those alkoxy groups of from 1 to 6 carbon atoms in a straight or branched chain configuration such as methoxy, ethoxy, propoxy, isopropoxy, butoxy, pentoxy, hexoxy, and the like.

The term "loweralkanoyl" is intended to include those alkanoyl groups of from 1 to 6 carbon atoms in a straight or branched chain configuration such as formyl, acetyl, propionyl, butyryl, pentanoyl, hexanoyl, and the like.

The term "halogen" is intended to include the halogen atoms, fluorine, chlorine, bromine, or iodine.

The term "acyl" is intended to include loweralkanoyl groups of from 1 to 6 carbon atoms of either a straight or branched configurations; substituted loweralkanoyl wherein the substituents, from 1 to 3, include cycloalkyl of from 3 to 6 carbon atoms, halogen, hydroxy, cyano, loweralkoxy, phenyl or substituted phenyl where the substituents, from 1 to 3, include halogen, hydroxy, cyano, nitro or loweralkoxy. The above structural formula is shown without a definitive stereochemistry. However, during the course of the synthetic procedures used to prepare such compounds, the products of such procedures can be a mixture of stereoisomers. In particular, the substituents of the stereoisomers at the 4″-, 4′-, 13-, 23-, 24-, and 25-positions may be oriented either α- or β- representing such groups being below or above the general plane of the molecule, respectively. In each such case both the α- and β- configurations are intended to be included within the ambit of this invention. In certain cases the term "epi" is used to distinguish the stereoisomer being of opposite configuration to the natural

compound at one specific asymetrical carbon atom.

PREPARATION OF STARTING MATERIALS

The ultimate starting materials for the compounds of this invention are the avermectin and milbemycin fermentation products defined in the background of the invention, in particular those containing a 23-hydroxy substituent which can be readily modified to the required intermediate. Avermectin B2a/B2b is a particularly useful and readily available fermentation product for this purpose, but others are not excluded from this reaction series. For this purpose the 4″-, 5-, and 7-hydroxy groups are protected as either the trialkylsilylethers or as phenoxyacetyl esters, advantagiously as the 4″,5-di-O-tert-butyldimethylsilyl-7-O-trimethylsilylether, and as 4″,5-di-O-phenoxyacetyl-7-O-trimethylsilylether. Reaction of the such protected avermectin compounds (II) with diethylaminosulfur trifluoride (generally referred to as DAST) at room temperature effected fluoridation as well as predominantly elimination to give the 23-fluoro-23-deoxyavermectin compounds (III) and 23-deoxy-Δ23,24-avermectin compounds (IV) respectively. Compound IV is differentiated from the naturally occurring avermectins in that it has a double bond at C23-24 that is once removed from the C21 dioxaspirane carbon compared to the avermectin A/B1 series. As a result this trisubstituted double bond is expected to be more reactive towards electrophilic reagents such as 3-chloroperoxybenzoic acid (MCPBA) and the like. Consequently epoxidation with MCPBA or other such peracid-like reagents predominantly affords the 23,24-oxides V in addition to some 14,15,23,24-bisoxide. In addition, reaction of selenium dioxide with IV produces the 24a-hydroxy VI.

PREPARATION OF COMPOUNDS

With this crucial intermediate (Scheme 1: compound V) available, these epoxides are reacted with various nucleophiles and the epoxides are hydrolized under acidic conditions. Upon subjecting epoxides V to aqueous hydrofluoroboric acid in ether, a rapid and unexpected rearrangement takes place to give the 1,3-dihydroxy product VII in addition to the expected 1,2-diol (scheme 2). This product VII exists mainly as the open form VIIB and subsequent oxidation at the C23 hydroxy function has led to fragmentation product X5 which is the avermectin structure devoid of C22-28. Addition of various nucleophiles to V have produced the adducts VIII and IX depending on the choice of reagents. For example addition of diethylaluminum cyanide to V gave the 24-cyano derivative VIII. Oxidation of VIIB with DMSO, oxalyl chloride leads to an open tri-carbonyl product (Scheme 3). Treatment of VIIB with BSTFA or 2,2-dimethoxypropane leads to products 4 or 5, respectively (Scheme 4).

A reaction sequence as described above for avermectin B2a can also be carried out with starting materials having as C-13 substituents a monooleandroside, a suitably protected hydroxy group, a hydrogen, halogen, alkoxy, alkanoyloxy or alkyl group, and products obtained by this variation of the above procedure all also meant to be included in the ambit of this invention.

## SCHEME 1

I

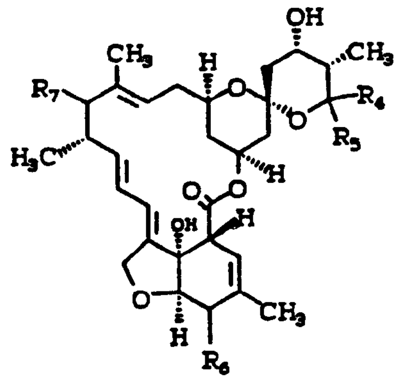

a) tBuSiMe$_2$Cl, imidazole, DMF

or PhOCH$_2$COCl, pyr, CH$_2$Cl$_2$

b) BSTFA, DMF

c) AcOH, THF, H$_2$O

a, b, c

II (Protected Avermectin compounds)

DAST

III IV

III

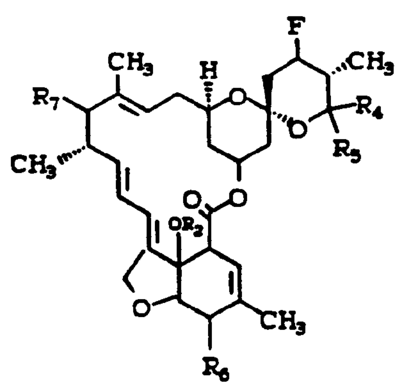

SCHEME 1 (CONT'D)

II

DAST

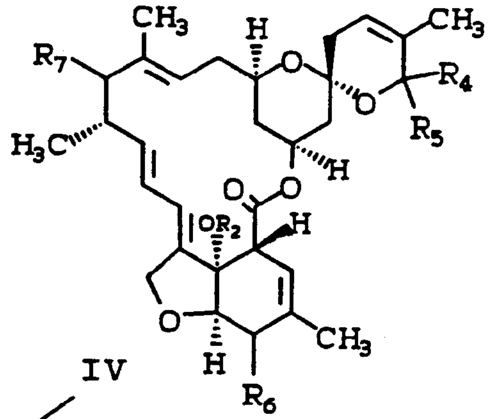

IV

$SeO_2$

VI

MCPBA

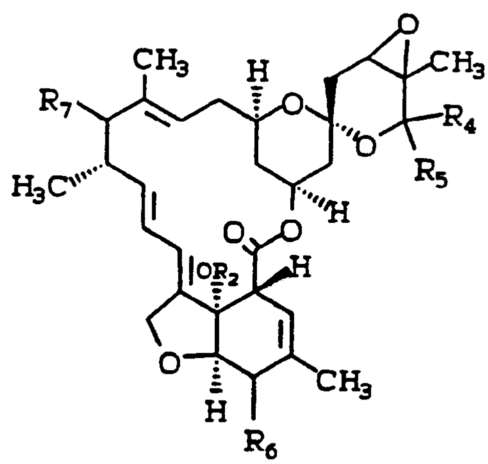

V

SCHEME 1 (CONT'D)

IV

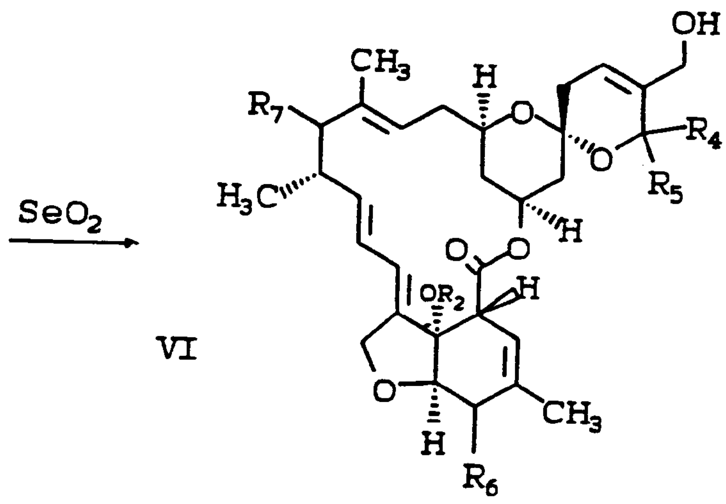


VI

## SCHEME 2

V

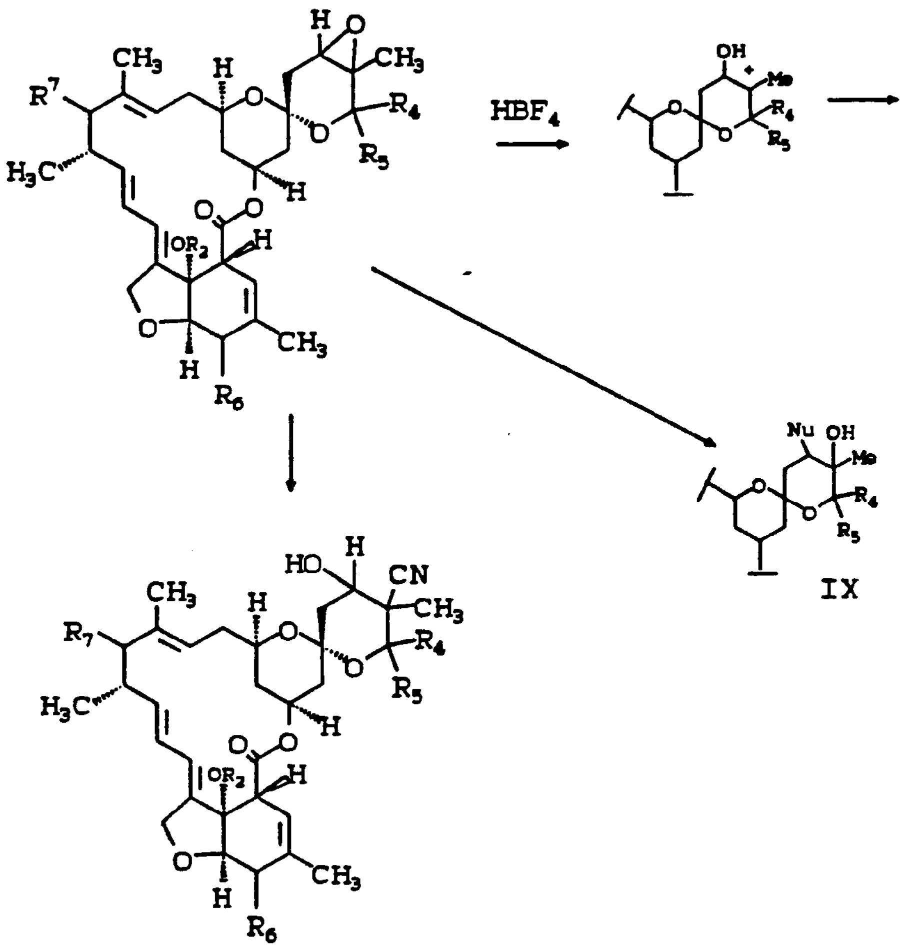


VIII

IX

SCHEME 2 (CONT'D)

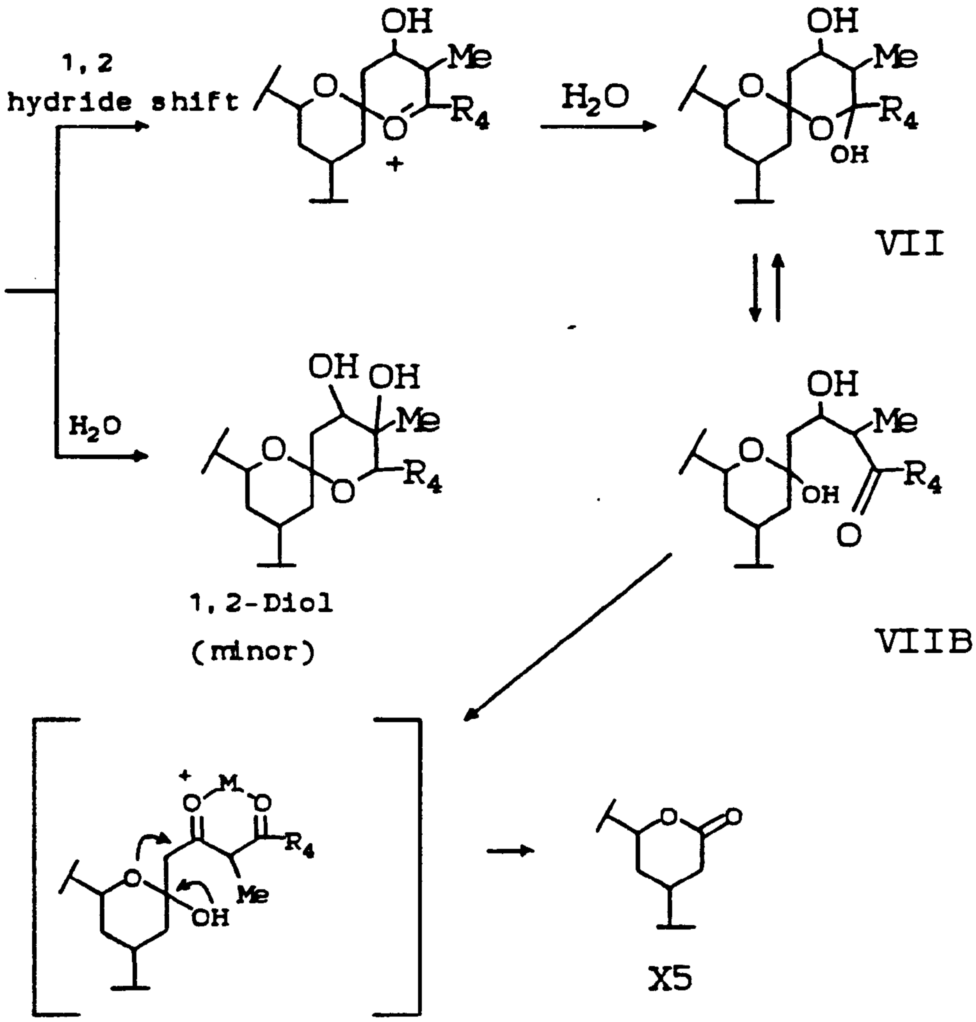


VII

1,2-Diol
(minor)

VIIB

X5

SCHEME 3

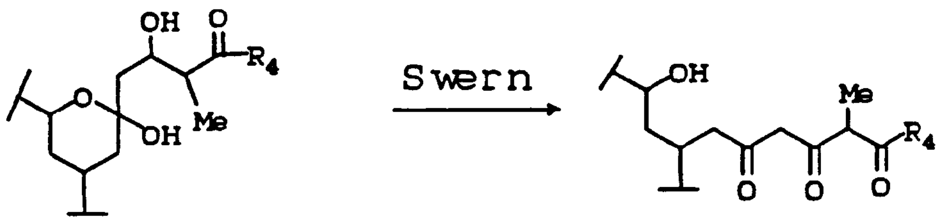


VIIB 3

SCHEME 4

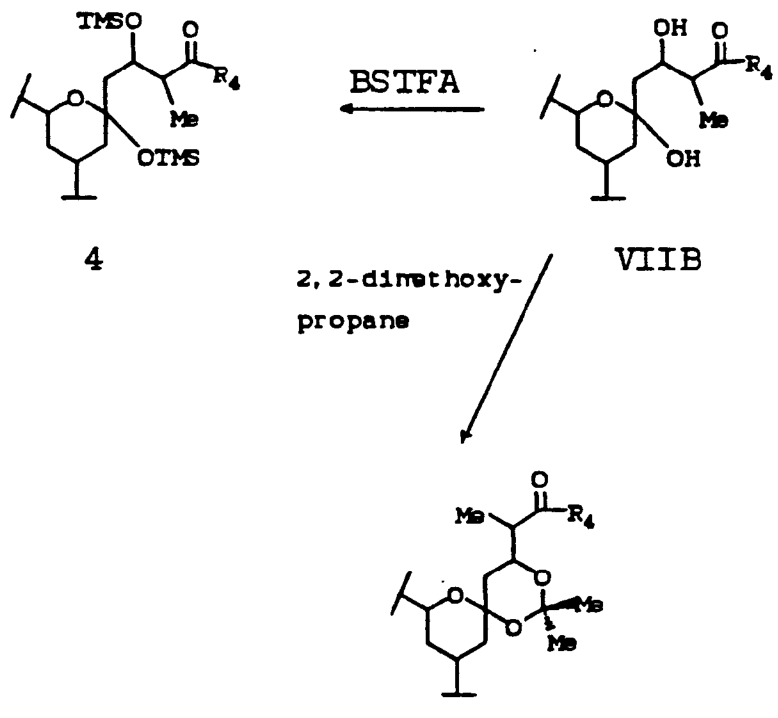


4 VIIB

5

The products obtained through the reaction sequence described above can be used as starting materials for further modifications in order to improve potency, safety, anthelmintic and insectcidal spectrum, stability, application formulations and other desirable properties for antiparasitic agents.

It is apparent that additional reaction sequences are required to prepare further compounds for the instant invention. Specifically, reactions are carried out at the 4″; 4′, 5, 13, 22, and 23-positions. Thus oxidation of the 4″- or 4′-hydroxy group, reductive amination of the thus produced 4″- or 4′-ketone to the 4″- or 4′-aminocompounds and their subsequent acylation is fully described in U. S. Patent No. 4,427,663.

During the oxidation and certain substitution reactions described above it is necessary to protect the 5-hydroxy group to avoid oxidation or substitution at that position. With this position protected reactions at the

4″- or 4′-positions may be carried out without affecting the remainder of the molecule. Subsequent to any of the above described reactions the protecting group may be removed and the unprotected product isolated. The protecting group employed is ideally one which may be readily synthesized, will not be affected by the reactions at the 4″- and 4′-positions and may be removed without affecting any other functionality of the molecule. One preferred type of protecting group for the avermectin type of molecule is the tri-substitiuted silyl group, preferably the trialkyl silyl group. One especially preferred example is the t-butyldimethylsilyl group. The reaction preparing the protected compound is carried out by reacting the hydroxy compound with the appropriately substituted silylhalide, preferably the silylchloride in an aprotic polar solvent such as methylene chloride, benzene, toluene, ethyl acetate, tetrahydrofuran, dimethylformamide and the like. In order to minimize side reactions, there is included in the reaction mixture a base to react with the acid halide released during the course of the reaction. Preferred amines are imidazole, pyridine, or triethylamine. The base is required in amounts equimolar to the amount of hydrogen halide liberated; however, generally several equivalents of the amine are employed. The reaction is stirred at from 0°C to the reflux temperature of the reaction mixture and is complete in from 1/2 to 16 hours. The silyl group is removed by stirring the silylated compound in methanol catalyzed by an acid preferably a sulfonic acid monohydrate such as p-toluenesulfonic acid monohydrate. The reaction is complete in about 1 to 12 hours at from 0 to 50°C. Alternatively, the silyl group may be removed by treatment of the silyl compound with anhydrous pyridine-hydrogen fluoride in tetrahydrofuran. The reaction is complete in from 3 to 24 hours at from 0 to 25°C.

Another useful modification of products obtained in the above reaction sequence are analogs in which the 22,23-double bond has been reduced to a single bond. The preferred catalyst for the selective hydrogenation of the 22,23-double bond is one having the formula:

$$[((R)_3P)_3RhY]$$

wherein

R is loweralkyl, phenyl, or loweralkyl substituted phenyl and Y is halogen.The reduction is completely described in U.S. Patent 4,199,569.

Other intermediates which are either used in the above reaction scheme or are prepared from the products of the above described reaction sequence involve the preparation of the monosaccharide. The processes which may be used to prepare the monosaccharide derivatives of the avermectin compounds are described in U.S. Patent 4,206,205. The reaction consists generally in treating the starting disaccharide with acid in an aqueous organic solvent mixture. Water concentration of from 0.1 to 20% by volume and acid concentrations of from about 0.01 to 0.1% will predominantly produce the monosaccharide, while acid concentrations from 1 to 10 % produce the aglycones. Another procedure for the preparation of the monosaccharide utilizes a 1% mineral acid solution in isopropanol at 20 to 40°C for from 6 to 24 hours. Mineral acids such as sulfuric, phosphoric, and the like may be employed.

The substituent at the 25- position of the avermecins is inert to the reaction conditions and the presence of alkyl groups, alkenyl groups, cycloalkyl groups, cycloalkenyl groups, aryl groups and the like at this position will have little affect on further chemical modifications of the avermectins.

All of the foregoing reactions carried out at the 4″-position of the avermectin can be carried out at the 4′-position of the monosaccharide to afford the correspondingly substituted monosaccharide derivatives.

## BIOLOGICAL ACTIVITIES OF THE INSTANT COMPOUNDS.

The novel compounds of this invention have significant parasiticidal activity as anthelmintics, ectoparasiticides, insecticides, and acaracides, in human and animal health and in agriculture.

The disease or group of diseases described generally as helminthiasis is due to infection of an animal host with parasitic worms known as helminths. Helminthiasis is a prevalent and serious economic problem in domesticated animals such as swine, sheep, horses, cattle, goats, dogs, cats, and poultry. Among the helminths the group of worms described as nematodes causes widespread and oftentimes serious infection in varios species of animals. The most common genera of nematodes infecting the animals referred to above are Haemonchus, Trichostrongylus, Ostertagia, Nematodirus, Cooperia, Ascaris, Bunostomum, Oesophagostomum, Chabertia, Trichuris, Strongylus, Trichonema, Dictocaulus, Capillaria, Heterakis, Toxocara, Ascaridia, Oxyuris, Ancylostoma, Uncinaria, Toxascaris, and Parascaris. Certain of these, such as Nematodirus, Cooperia, and Oesophagostomum attack primarily the intestinal tract while others, such as Haemonchus and Ostertagia, are more prevalent in the stomach while still others such as Dictocaulus are found in the lungs. Still other parasites may be located in other tisues and organs of the body such as the heart and blood vessels, subcutaneous and lymphatic tissue and the like. The parasitic infections known as helminthiasis lead to anemia, malnutrition, weakness, weight loss, severe damage to the walls of the intestinal tract and other tissues and organs and, if left untreated, may result in the death of the infected host. The avermectin compounds of this invention have

unexectedly high activity against Dirofilaria in dogs, Nematospiroides, Syphacia, Aspiculuris in rodents, anthropod ectoparasites of animals and birds such as ticks, mites, lice, fleas, blowfly, in sheep Lucilia sp., biting insects and such migrating dipterous larvea Hypoderma sp. in cattle, Gastrophilus in horses, and Cuterebra sp. in rodents.

The instant compounds are also useful against parasites which infect humans. The most common genera of parasites of the gastro-intestinal tract of man are Ancylostoma, Necator, Ascaris, Strongyloides, Trichinella, Capillaria, Trichuris, and Enterobius. Other medically important genera of parasites which are found in the blood or other tissues and organs outside the gastrointestinal tract are the filiarial worms such as Wuchereria, Brugia, Onchocerca and Loa, Dracunculus and extra-intestinal stages of the intestinal worms Strongyloides. and Trichinella. The compounds are also of value against arthropods parasitizing man, biting insects and other dipterous pests causing annoyance to man.

The compounds are also active against household pests such as the cockroach, Blatella sp., clothes moth, Tineola sp., carpet beetle, Attagenus sp., and the housefly Musca domestica.

The compounds are also useful against insect pests of stored grains such as Tribolium sp., Tenebrio sp. and of agricultural plants such as spider mites (Tetranychus sp.) aphids (Acyrthiosiphon sp.); against migratory orthopterans such as locusts and immature stages of insects living on plant tissue. The compounds are useful as a nematocide for the control of soil nematodes and plant parasites such as Meloidogyne spp. which may be of importance in agriculture.

These compounds may be administered orally in a unit dosage form such as a capsule, bolus or tablet, or as a liguid drench where used as an anthelmintic in mammals. The drench is normally a solution, suspension or dispersion of the active ingredient usually in water together with a suspending agent such as bentonite and a wetting agent or like excipient. Generally, the drenches also contain an antifoaming agent. Drench formulations generally contain from about 0.001 to 5% by weight of the active compound. Preferred drench formulations may contain from 0.01 to 0.1% by weight active compound. The capsules or boluses are comprised of the active ingredient admixed with a carrier vehicle such as starch, talc, magnesium stearate, or di-calcium phosphate.

Where it is desired to administer the avermectin derivatives in a dry, solid unit dosage form, capsules, boluses, or tablets containing the desired amount of active compound usually are employed. The dosage forms are prepared by intimately and uniformly mixing the active ingredients with suitable finely divided diluents, fillers, disintegrating agents, and/or binders such as starch, lactose, talc, magnesium stearate, vegetable gums and the like. Such unit dosage formulations may be varied widely with respect to their total weight and content of antiparasitic agent depending upon factors such as the type of host animal to be treated, the severity and type of the infection and the weight of the host.

When the active compound is to be admnistered via the animal feedstuff, it is intimately dispersed in the feed or used as a top dressing or in the form of pellets which may then be added to the finished feed or optionally fed separately. Alternatively, the antiparasitic compounds of our invention may be administered to the animals parenterally, for example, by intraruminal, intramuscular, intratracheal, or subcutaneous injection in which the active ingredient is dissolved or dispersed in a liquid carrier vehicle. For parenteral administration, the active material is suitably admixed with an acceptable vehicle, preferably of the vegetable oil variety such as peanut oil, cotton seed oil, and the like. Other parenteral vehicles such as organic preparations using solketal, glycerol formal, and aqueous parenteral formulations are also used. The active avermectin compound or compounds are dissolve or suspended in the parenteral formulation for administration; such formultions generally contain from 0.005 to 5% by weight of the active compound.

Although the antiparasitic agents of this invention find their primary use in the treatment and/or prevention of helminthiasis,they are also useful in the prevention and treatment of diseases caused by other parasites, for example, arthropod parasites such as ticks, lice, fleas, mites, and other biting insects in domesticated animals and poultry. They are also effective in treatment of parasitic diseases that occur in other animals including humans. The optimum amount to be employed for the best results will, of course, depend upon the particular compound employed, the species of animal to be treated and the type and severity of parasitic infection or infestation. Generally good results are obtained with our novel compounds by the oral administration of from about 0.001 to 10 mg per kg of animal body weight, such total dose being given at one time or in divided doses over a relatively short period of time such as 1-5 days. With the preferred compounds of the invention, excellent control of such parasites is obtained in animals by administratering from about 0.025 to 0.5 mg per kg of body weight in a single dose. Repeat treatments are given as required to combat re-infections and are dependent upon the species of parasite and the husbandry techniques being employed. The techniques for administering these materials to animals are known to those skilled in the veterinary field. When the compounds described herein are asministered as a component of the feed of the animals, or dissolved or suspended in the drinking water, compositions are povided in which the active compound or compounds are intimately dispersed in an

inert carrier or diluent. By inert carrier is meant one that will not react with the antiparasitic agent and one that may be administered safely to animals. Preferably, a carrier for feed administration is one that is, or may be, an ingredient of the animal ration.

Suitable compositions include feed premixes or supplements in which the active ingredient is present in relatively large amounts and which are suitable for the direct feeding to the animal or for addition to the feed either directly or after an intermediate dilution or blending step. Typical carriers or diluents suitable for such compositions include, for example, distillers' dried grains, corn meal, citrus meal, fermentaion residues, ground oyster shells, wheat shorts, molasses solubles, corn cob meal, edible bean mill feed, soya-grits, crushed limestone and the like. The active avermectin compounds are intimately dispersed throughout the carrier by methods such as grinding, stirring, milling, or tumbling. Compositions containing from about 0.005 to 2.0% weight of the active compound are particularly suitable as feed premixes. Feed supplements, which are fed directly to the animal, contain from about 0.002 to 0.3% by weight of the active compounds.

Such supplements are added to the animal feed in an amount to give the finished feed the concentration of active compound desired for the treatment and control of parasitic diseases. Although the desired concentration of the active compound will vary depending upon the factors previously mentioned as well as upon the particular avermectin derivative employed, the compounds of this invention are usually fed at concentrations of between 0.00001 to 0.002% in the feed in order to achieve the desired antiparasitic result.

In using the compounds of this invention, the individual avermectin components may be prepared and used in that form. Alternatively, mixtures of two or more of the individual avermectin components may be used, or other active compounds not related to the compounds of this invention.

The compounds of this invention are also useful in combatting agricultural pests that inflict damage upon crops while they are growing or in storage. The compounds are applied using known techniques as sprays, dusts, emulsions and the like, to the growing or stored crops to effect protection from such agricultural pests.

The following examples are provided in order that this invention might be more fully understood; they are not to be construed as limitative of the invention.

The avermectin derivatives prepared in the following examples are generally isolated as amorphous solids and not as crystalline solids. They are thus characterized analytically using techniques such as mass spectrometry, nuclear magnetic resonance spectrometry and the like. Being amorphous, the compounds are not characterized by sharp melting points, however, the chromatographic and analytical methods employed indicate that the compounds are pure.

Certain abbreviations are used in the following examples which have the following meanings.

BSTFA - Bis(trimethylsilyl)trifluoroacetamide
DAST - Diethylamino sulfur trifluoride
MCPBA - 3-Chloroperoxy benzoic acid
PLC - Preparative layer chromatography
NMR - Nuclear magnetic resonance
DMF - N,N-Dimethyl formamide
THF - Tetrahydrofuran

## Example 1

5-O-t-Butyldimethylsilyl-Avermectin B2a/B2b and 4″,5-Di-O-t-Butyldimethylsilyl-Avermectin B2a/B2b.

To a solution of 58.2 g (65 mmol) of dried avermectin B2a/B2b in 400 mL of sieve-dried DMF and 30 mL of freshly distilled triethylamine was added a solution of 29.8 g (198 mmol, 3 equiv.) of t-butyldimethylsilyl chloride in 200 mL of dichloromethane. The mixture was stirred at room temperature 16 hours then poured into ice water and extracted with dichloromethane. The organic phases were combined and washed with water, brine, and dried over magnesium sulfate. Evaporation of the solvent afforded an oil which was purified by silica gel high performance liquid chromatography using 20% ethyl acetate-hexane to yield 34.2 g of 4″,5-Di-O-t-butyldimethylsilylavermectin B2a/B2b and 5 g of 5-O-t-butyldimethylsilylavermectin B2a characterized by their NMR and mass spectra.

## Example 2

4″,5-Di-O-t-butyldimethylsilyl-7-O-trimethylsilylavermectin B2a/B2b

A solution of 3.3 g of 4″,5-Di-O-t-butyldimethylsilylavermectin B2a/B2b in 10 mL of DMF was stirred with 20 mL of BSTFA at room temperature for 50 h. The volatiles were removed in vacuo and the residual solid was

taken up in dichloromethane and filtered through a silica gel column with 10 % ethyl acetate-hexane . The solvents were removed to afford 3.3 g of 4″,5-Di-O-t-butyldimethylsilyl-7,23-di-O-trimethylsilylavermectin B2a/B2b. This was dissolved in 200 mL of 9:1 THF:water and 18 mL of glacial acetic acid was added. After 24 h, the THF was removed in vacuo and sodium bicarbonate was added to neutralize the acid and the organic product was extracted with ethyl acetate. Flash chromatographic purification through a silica gel column afforded 2.8 g of 4″,5-di-O-t-butyldimethylsilyl-7-O-trimethylsilylavermectin B2a/B2b characterized by its NMR and mass spectra.

Exemple 3

4″,5-Di-O-t-butyldimethylsilyl-7-O-trimethylsilyl-22-hydro-Δ-23,24-avermectin B1a/B1b and 4″,5-Di-O-t-butyldimethylsilyl-7-O-trimethylsilyl-22-hydro-23-fluoroavermectin B1a/B1b

To a stirred solution of 2.29 g of 4″,5-di-O-t-butyldimethylsilyl-7-O-trimethylsilylavermectin B2a/B2b in 10 mL of dichloromethane at room temperature was added 0.55 mL of DAST. After 15 min the solution was flash chromatographed through 150 g of silica gel with 10% ethyl acetate-dichloromethane. The initial separation gave 1.5 g of a mixture of the titled compounds which were further purified by PLC to afford 1.22 g of 4″,5-di-O-t-butyldimethylsilyl-7-O-trimethylsilyl-22-hydro-Δ-23,24-avermectin B1a/B1b and 131 mg of 4″,5-di-O-t-butyldimethylsilyl-7-O-trimethylsilyl-22,23-dihydro-23-fluoroavermectin B1a/B1b each characterized by their NMR and mass spectra.

Example 4

4″,5-Di-O-phenoxyacetyl-avermectin B2a/B2b.

To a solution of 3 g of avermectin B2a/B2b in 30 mL of dichloromethane and 0.85 mL of pyridine at 0°C was added dropwise a solution of 1.41 mL of phenoxyacetyl chloride in 30 mL of dichloromethane. After completion of addition, the reaction mixture was stirred at 20°C for 1 h. The mixture was then added to 75 mL of ice-water and extracted with ether. The combined ether extracts were dried ($MgSO_4$) and evaporated to yield 4.3 g of products. Chromatographic separation on silica gel afforded 3.1 g of titled compound characterized by its NMR and mass spectra.

Example 5

4″,5-Di-O-phenoxyacetyl-7,23-di-O-trimethylsilylavermectin B2a/B2b

To 3.1 g of 4″,5-di-O-phenoxyacetylavermectin B2a/B2b in 10 mL of DMF was added 20 mL of BSTFA. The mixture was stirred for 24 h at room temperature before the solvent and excess BSTFA were removed in vacuo. Flash chromatographic purification on silica pel gave 2.9 g of titled product characterized by its NMR and mass spectra.

Example 6

4″,5-Di-O-phenoxyacetyl-7-O-trimethylsilylavermectin B2a/B2b

To a solution of 3.39 g of 4″,5-di-O-phenoxyacetyl-7,23-di-O-trimethylsilyavermectin B2a/B2b in 270 mL of tetrahydrofuran was added 30 mL of water and 30 mL of acetic acid. The mixture was stirred at room temperature for 44 h before the THF was removed in vacuo. The acetic acid was neutralized by the addition of aqueous sodium bicarbonate solution and the product was extracted with ethyl acetate. The product obtained after evaporation of the solvent was purified by column chromatography and characterized by NMR and mass spectroscopy.

Example 7

4″,5-Di-O-phenoxyacetyl-7-O-trimethylsily-22-hydro-Δ23,24-avermectin B1a/B1b

To a solution of 304 mg of 4″,5-di-O-phenoxyacetyl-7-O-trimethylsily-avermectin B2a/B2b in 2.5 mL of dichloromethane was added 70 microliters of DAST. After 12 min the mixture was eluted on three 1 mm thick

silica gel plates with 33% ethyl acetate-hexane. The major band, Rf= 0.5, was removed and extracted to afford 130 mg of the titled compound characterized by its NMR and mass spectra.

Example 8

4″,5-Di-O-phenoxacetyl-7-O-trimethylsilyl-22-hydro-Δ-23,24-24a-hydroxyavermectin B1a/B1b

To a stirred solution of 100 mg of 4″,5-Di-O-phenoxyacetyl-7-O-trimethylsilyl-22-hydro-Δ-23,24-avermectin B1a/B1b in 1 mL of dichloromethane was added 10 mg of selenium dioxide, 5 mg of salicyclic acid, and 110 microliters of a 3 M solution of tert-butyl-hydroperoxide in toluene. After 24 h at 20°C, another 10 mg of selinium dioxide and 90 microliters of a 3 M solution of tert-butylhydro-peroxide in toluene is added and the mixture stirred an additional 16 h. The mixture was then separated on a preparative silica gel plate to afford 20 mg of the titled product characterized by its NMR and mass spectra.

Example 9

4″,5-Di-O-t-butyldimethylsilyl-7-O-trimethylsilyl-22-hydro-24-dehydro-23,24-epoxyavermectin B1a/B1b

To a solution of 1.2 g of 4″,5-Di-O-t-butyldimethylsilyl-7-O-trimethylsilyl-22-hydro-Δ-23,24-avermectin B1a/B1b in 10 mL of dichloromethane was added a solution of 221 mg of 80% MCPBA in dichloromethane. After 30 min 300 mg of dimethyl sulfide was added and the reaction mixture was stirred another 30 min. The reaction mixture was then concentrated in vacuo and the residue was dissolved in ether and washed successively with aqueous sodium bicarbonate and brine. The ether was removed and the solid residue was flash chromatographed to afford 447 mg of 4″,5-di-O-t-butyldimethylsilyl-7-O-trimethylsilyl-22-hydro-23,24-epoxyavermectin B1a/B1b characterized by its NMR and mass spectra.

Example 10

4″,5-Di-O-phenoxyacetyl-7-O-trimethylsilyl-22-hydro-23.24-epoxyavermectin B1a/B1b

The procedure used in example 9 was applied to 4″,5-Di-O-phenoxyacetyol-7-O-trimethylsily-22-hydro-Δ-23,24-avermectin B1a/B1b to afford the title compound in 30-35% yield as a separable mixture of alpha and beta epoxides characterized by their NMR and mass spectra.

Example 11

22-Hydro-23,24-epoxy-avermectin B1a/B1b

To 72 mg of 4″,5-di-O-t-butyldimethylsilyl-7-O-trimethylsilyl-22-hydro-23,24-epoxyavermectin B1a/B1b in 2 mL of THF was added 5 mL of hydrogen fluoride in pyridine-THF ( prepared by diluting 20 mL of commercial HF-pyridine complex with 60 mL of distilled pyridine and 120 mL of THF). After 2 days at room temperature, the HF solution was neutralized with aqueous sodium bicarbonate. The product was extracted with ether and chromatographic purification afforded 14.8 mg of title compound characterized by its NMR and mass spectra.

Example 12

4″,5-Di-O-phenoxyacetyl-7-O-trimethylsily-22-hydro-23-hydroxy-24-cyano-avermectin B1a/B1b

To 83 mg of 4″,5-di-O-phenoxyacetyl-7-O-trimethylsily-22-hydro-23,24-epoxyavermectin B1a/B1b was added 1 mL of a 1.0 M solution of diethylaluminum cyanide in toluene. The solution was stirred at room temperature for 1h 20 min before 3 mL of a 2.0 M solution of HCl was added dropwise to quench the reaction. The mixture was extracted with ether and the dried ($MgSO_4$) extracts were concentrated in vacuo to afford 72 mg of products. Preparative layer chromatographic purification afforded-25 mg of the title compound characterized by its NMR and mass spectra.

Example 13

7-O-Trimethylsily-22-hydro-23-hydroxy-24-cyano-avermectin B1a/B1b

To 70 mg of 4″,5-di-O-phenoxyacetyl-7-O-trimethylsilyl-22-hydro-23-hydroxy-24-cyano-avermectin B1a/B1b in 2 mL of THF at 0°C was added 0.300 mL of 3.0 M methylmagnesium bromide in THF. After 5 min, 2 mL of a saturated solution of aqueous ammonium chloride was added. The mixture was extracted with ether. Removal of the solvent followed by preparative layer chromatographic purification afforded 35 mg of the title compound characterized by its NMR and mass spectra.

Example 14

22-Hydro-25-hydroxy-24-cyanoavermectin B1a/B1b

To 28 mg of 7-O-trimethylsilyl-22-hydro-23-hydroxy-24-cyano-avermectin B1a was added 2 mL of freshly distilled THF followed by 3 mL of hydrogen fluoride in pyridine-THF ( prepared by diluting 20 mL of commercial HF-pyridine complex with 60 mL of distilled pyridine and 120 mL of THF). the mixture was stirred for 21 h and the HF was neutralized with aqueous sodium bicarbonate. The product was extracted with ether and chromatographic purification afforded 20 mg of 22-hydro-23-hydroxy-24-cyanoavermectin B1a/B1b characterized by its NMR and mass pectra.

Example 15

4″,5-Di-O-t-butyldimethylsilyl-7-O-trimethylsilyl-25-hydroxy-25-dehydro-avermectin B2a/B2b and4″,5-Di-O-t-butyldimethylsilyl-7-O-trimethylsilyl-24- hydroxy-24-dehydro-avermectin B2a/B2b

To 8.8 g of 4″,5-di-O-t-butyldimethylsilyl-7-O-trimethylsilyl-22-hydro-24-dehydro-23,24-epoxy-avermectin B1a/B1b in 100 mL of ether in a separatory funnel was added 8 mL of a 48% aqueous $HBF_4$ solution and the mixture was shaken for 1 min. A solution of 10 g of sodium carbonate in 100 mL of water was then added to neutralize the acid. The ether layer was then separated, dried ($MgSO_4$), and concentrated to dryness. The residual solid was flash chromatographed on silica gel to yield 4.5 g of 4″,5-di-O-t-butyldimethylsilyl-7-O-trimethylsilyl-25-hydroxy-avermectin B2a/B2b and 1 g of 4″,5-di-O-t-butyldimethylsilyl-7-O-trimethylsilyl-24-hydroxy-24-dehydro-avermectin B2a/B2b characterized by their NMR and mass spectra.

Example 16

4″,5-Di-O-phenoxyacetyl-7-O-trimethylsilyl-25-hydroxy-avermectin B2a/B2b and 4″,5-Di-O-phenoxyacetyl-7-O-trimethylsilyl-24-hydroxy-avermectin B2a/B2b

To 163 mg of 4″,5-Di-O-phenoxyacetyl-7-O-trimethylsilyl-22-hydro-23,24-epoxy-avermectin B1a/B1b (major alpha epoxide isomer) in 8 mL of ether was added 0.075 mL of 48% $HBF_4$. After 35 min, the reaction was quenched with sodium bicarbonate solution. The ether extracts were combined and concentrated to afford 170 mg of products. Chromatographic purification gave 83 mg of 4″,5-di-O-phenoxyacetyl-7-O-trimethylsilyl-25-hydroxy-avermectin B2a/B2b and 15 mg of 4″,5-di-O-phenoxyacetyl-7-O-trimethylsilyl-24-hydroxy-avermectin B2a/B2b characterized by their NMR and mass spectra.

Example 17

25-Hydroxy-avermectin B2a/B2b

The procedure used in example 14 was employed to desilylate 105 mg of 4″,5-di-O-t-butyldimethylsilyl-7-O-trimethylsilyl-25-hydroxy-avermectin B2a/B2b to yield 32 mg of the title compound characterized by its NMR and mass spectra.

Example 18

24-Hydroxy-avermectin B2a/B2b

The procedure used in example 14 was employed to desilylate 38 mg of 4″,5-di-O-t-butyldimethylsilyl-7-O-trimethylsilyl-24-hydroxy-24-dehydro-avermectin B2a/B2b to yield 14 mg of the title compound characterized by its NMR and mass spectra.

Example 19

Structure 5, C21-23 Acetonide

To a solution of 100 mg of 4″,5-di-O-t-butyldimethylsilyl-7-O-trimethylsilyl-25-hydroxy-avermectin B2a/B2b in 2 mL of 2,2-dimethoxy-propane was added 50 mg of pyridinium tosylate. The mixture was stirred at room temperature for 18 h and then the excess volatile liquid was removed in vacuo. The residue was purified by preparative layer chromatography to afford 48 mg of compound X2 having silyl groups at the 4″,5 and 7 positions. This was desilylated with 2 mL of HF-pyridine-THF as in Example 14 (except the reaction was over in 18 h) to yield 25 mg of purified product characterized by its NMR and mass spectra.

Example 20

Structure 3

To a solution of 0.36 mL of dimethyl sulfoxide in 4 mL of dry dichloromethane cooled to -78°C was added 0.28 mL of oxalyl chloride. After 2 min, a solution of 200 mg of 4″,5-di-O-t-butyldimethylsilyl-7-O-trimethylsilyl-25-hydroxy-avermectin B2a/B2b in 1 mL of dichloromethane was added dropwise. After 1 h, 1 mL of triethylamine was added and the reaction was stirred an additional 1.5 h. Several mL of a saturated ammonium chloride solution was then added to quench the reaction. The organic products were extracted from the aqueous phase with ether. Preparative layer chromatography afforded 20 mg of 4″,5-di-O-t-butyldimethylsilyl-7-O-trimethylsilyl-23-oxo-25-hydroxy-avermectin B2a/B2b which has an Rf of 0.5 in 20% ethyl acetate-hexane on silica gel TLC. The reaction was repeated to obtain another 21 mg of the above product and 41 mg of this product was desilylated in 2 mL of HF-pyridine-THF as in example 14 to yield 6.6 mg of the title compound characterized by its NMR and mass spectra.

Example 21

Structure X5

To a solution of 840 mg of 4″,5-di-O-t-butyldimethylsilyl-7-O-trimethylsilyl-25-hydroxy-avermectin B2a/B2b and 300 mg of 4-methylmorpholine-N-oxide in 15 mL of dichloromethane was added 100 mg of tetrapropylammonium penuthenate. After 20 min, 1 g of silica gel was added and the mixture was filtered through a short column of silica gel with dichloromethane and ethyl acetate. The residue obtained after concentration of the filtrate was purified by chromatography to afford 60 mg of structure X5 containing the silyl groups as in the starting material characterized by its NMR and mass spectra.

Example 22

22-Hydro-Δ-23,24-24a-hydroxyavermectin B1a/B1b

To 99 mg of 4″,5-Di-O-phenoxyacetyl-7-O-trimethylsilyl-22-hydro-Δ-23,24-24a-hydroxyavermectin B1a/B1b in 2 mL of THF at 0°C was added 0.300 mL of 3 M methylmagnesium bromide in ethyl ether. After 5 min the reaction mixture was quenched with 5 mL of sodium bicarbonate solution and the product, 7-O-trimethylsilyl-22-hydro-Δ-23,24-24a-hydroxyavermectin B1a/B1b, was isolated by extraction with ether to afford 64 mg of glassy foam. This was desilylate using the procedure of example 14 to yield 31 mg of 22-hydro-Δ-23,24-24a-hydroxyavermectin B1a/B1b characterized by its NMR and mass spectra.

Example 23

22,23-Dihydro-23-fluoroavermectin B1a/B1b

Treatment of 33 mg of 4″,5-Di-O-t-butyldimethylsilyl-7-O-trimethylsilyl-22-hydro-23-fluoroavermectin B1a/B1b with 2 mL of HF-pyridine in THF according to the procedure of example 14 gave 18.2 mg of the titled product characterized by its NMR and mass spectra.

Example 24

22-Hydro-Δ-23,24-avermectin B1a/B1b

Treatment of 100 mg of 4″,5-di-O-t-butyldimethylsilyl-7-O-trimethylsilyl-22-hydro-Δ-23,24-avermectin B1a/B1b with 4 mL of HF-pyridine in THF according to the procedure of example 14 gave 50 mg of the titled product characterized by its NMR and mass spectra.

Example 25

5-O-t-Butyldimethylsilyl-4″-oxoavermectin B2a/B2b

To a solution containing 9.1 ml of oxalyl chloride in 230 ml of dry methylene chloride stirred at -60°C is added 15 ml of dry dimethylsulfoxide dissolved in 120 ml of dry methylene chloride during 15 min. Then a solution of 46.5 g of 5-O-t-butyldimethylsilylavermectin B2a/B2b dissolved in 230 ml of dry methylene chloride is added over a period of 15 minutes while maintaining the temperature at -60°C. The reaction mixture is stirred at this temperature for 30 minutes when 65 ml of dry triethylamine is added. The mixture is stirred for 5 additional minutes at -60°C, and then the cooling bath is removed and the reaction mixture is allowed to come to ambient temperature. After addition of water the reaction product is extracted with methylene chloride, the extract is washed with water, dried and concentrated in vacuo to a yellow foam. Flash column silica gel chromatographic purification provides 5-O-t-butyldimethylsilyl-4″-oxoavermectin B2a/B2b.

Example 26

4″-epi-Amino-5-O-t-butyldimethylsilsyl-4″-deoxyavermectin B2a/B2b

For the reductive amination 12 mg of sodium cyanoborohydride is added to a solution of 200 mg of 5-O-t-butyldimethylsilyl-4″-oxoavermectin B2a/B2b (from Example 25) and 160 mg of ammonium acetate in 3 ml of methanol, and the reaction mixture is stirred at room temperature for 1 hour. Then it is poured into aqueous $Na_2CO_3$ solution, and the organic products are extracted with ethyl acetate. The extract is washed with water, dried, and concentrated in vacuo to a yellow oil. Preparative silica gel layer chromatography with 98:2 methylene chloride-methanol solvent gives 4″-epi-amino-5-O-t-butyldimethylsilyl-4″-deoxyavermectin B2a/B2b as a light foam, which is characterized by its mass and NMR spectra.

Example 27

5-O-t-Butyldimethylsilyl-4″-epi-acetylamino-4″-deoxyavermectin B2a/B2b

A sample of 100 mg of 4″-epi-amino-5-O-t-butyldimethylsilyl-4″-deoxyavermectin B2a/B2b (from Example 26) is dissolved in 2 mL of acetic anhydride and stirred for 1 h at room temperature. The solvent is then removed in vacuo and the residue is purified by preparative silica gel thick layer chromatography to yield 4″-epi-acetylamino-5-O-tert-butyldimethylsilyl-4″-deoxyavermectin B2a/B2b as a light yellow foam, which is characterized by its mass and NMR spectra.

Example 28

5-O-tert-Butyldimethylsilyl-7-O-trimethylsilyl-4″-deoxy-4″-epi-acetylaminoavermectin B2a/B2b

To a stirred solution of 100 mg of 4″-epi-acetylamino-5-O-tert-butyldimethylsilyl-4″-deoxyavermectin B2a/B2b in 1 mL of dry DMF is added 1 mL of BSTFA. After 24 h the solvent is removed in vacuo and the residual

solid is treated with 5 mL of 9:1:1 THF:acetic acid:water for 44 h as in example 6. The solution is then neutralized with sodium bicarbonate and the product is extracted with ethyl acetate. Purification by preparative silica gel thick layer chromatography gives 5-O-tert-butyldimethylsilyl-7-O-trimethylsilyl-4″-deoxy-4″-epi-acetylaminoavermectin B2a/B2b which is characterized by nuclear magnetic resonance and mass spectra.

Example 29

4″-epi-Acetylamino-4″-deoxy-5-O-tert-butyldimethylsilyl-7-O-trimethylsilyl-22-hydro-Δ-23,24-avermectin B1a/B1b and 4″-epi-Acetylamino-4″-deoxy-5-O-tert-butyldimethylsilyl-7-O-trimethylsilyl-22,23-dihydro-23-fluoroavermectin B1a/B1b

A solution of 5-O-tert-butyldimethylsilyl-7-O-trimethylsilyl-4″-deoxy-4″-epi-acetylaminoavermectin B2a/B2b in dichloromethane is treated with DAST as in Example 3 to give 4″-epi-acetylamino-4″-deoxy-5-O-tert-butyldimethylsilyl-7-O-trimethylsilyl-22-hydro-Δ-23,24-avermectin B1a/1b and 4″-epi-acetylamino-4″-deoxy-5-O-tert-butyldimethylsilyl-7-O-trimethylsilyl-22,23-dihydro-23-fluoroavermectin B1a/1b after purification and characterization by their NMR and mass spectra.

Example 30

4″-epi-acetylamino-4″-deoxy-22-hydro-Δ-23,24-avermectin B1a/B1b and 4″-epi-acetylamino-4″-deoxy-22,23-dihydro-23-fluoroavermectin B1a/B1b

Treatment of 4″-epi-acetylamino-4″-deoxy-5-O-tert-butyldimethylsilyl-7-O-trimethylsilyl-22-hydro-Δ-23,24-avermectin B1a/B1b and 4″-epi-acetylamino-4″-deoxy-5-O-tert-butyldimethylsilyl-7-O-trimethylsilyl-22,23-dihydro-23-fluoroavermectin B1a/1b from preparation E with HF-pyridine-THF as in Example 11 give 4″-epi-acetylamino-4″-deoxy-22-hydro-Δ-23,24-avermectin B1a/1b and 4″-epi-acetylamino-4″-deoxy-22,23-dihydro-23-fluoroavermectin B1a/B1b which are characterized by their NMR and mass spectra.

Example 31

4″-Deoxy-4″-epiacetylamino-22-hydro-23,24-epoxyavermectin B1a/B1b

To a solution of 100 mg of 4″-epi-acetylamino-4″-deoxy-22-hydro-Δ-23,24-avermectin B1a/B1b in 5 mL of dichloromethane is added a solution of 22 mg of 80% m-chloroperbenzoic acid in 1 mL of dichloromethane. After 30 min 30 mg of dimethyl sulfide is added and the reaction mixture is stirred another 30 min. The reaction mixture is then concentrated in vacuo and the residue is dissolved in ether and washed successively with aqueous sodium bicarbonate and brine. The ether is removed and the solid residue is purified by PLC to yield 4″-deoxy-4″-epiacetylamino-22-hydro-23,24-epoxyavermectin B1a/B1b characterized by its NMR and mass spectra.

Example 32

4″-Deoxy-4″-epiacetylamino-24-cyanoavermectin B2a/B2b

To 80 mg of 4″-deoxy-4″-epiacetylamino-23,24-epoxyavermectin B1a/B1b is added 3 mL of a 1.0 M solution of diethylaluminum cyanide in toluene. The solution is stirred at room temperature for 1h 20 min before 3 mL of a 2.0 M solution of HCl is added dropwise to quench the reaction. The mixture is extracted with ether and the dried ($MgSO_4$) extracts are concentrated in vacuo to afford the products. Preparative layer chromatographic purification affords the title compound characterized by its NMR and mass spectra.

Example 33

4″-Deoxy-4″-epiacetylamino-24-hydroxyavermectin B2a/B2b and 4″-Deoxy-4″-epiacetylamino-25-hydroxyavermectin B2a/B2b

To 100 mg of 4″-deoxy-4″-epiacetylamino-23,24-epoxyavermectin B1a/B1b in 8 mL of ether was added 0.075 mL of 48% $HBF_4$. After 5 min, the reaction is quenched with sodium bicarbonate solution. The ether extracts are combined and dried over magnesium sulfate. The mixture is then filtered and concentrated in vacuo.

The residual products are separated by PLC to afford the titled products characterized by their NMR and mass spectra.

Example 34

4″-Epi-acetylamino-4″-deoxy-22-hydro-Δ-23,24-24a-hydroxyavermectin B1a/B1b

To a stirred solution of 100 mg of 4″-epi-acetylamino-4″-deoxy-22-hydro-Δ-23,24-avermectin B1a/B1b in 1 mL of dichloromethane is added 10 mg of selenium dioxide, 5 mg of salicyclic acid, and 110 microliters of a 3 M solution of tert-butylhydroperoxide in toluene. After 24 h at 20°C, another 10 mg of $SeO_2$ and 90 microliters of a 3 M solution of tert-butylhydroperoxide in toluene is added and the mixture is stirred an additional 16 h. The mixture is then separated on a preparative silica gel plate to afford the titled product characterized by its NMR and mass spectra.

**Claims**

1. A compound having the following structural formula:

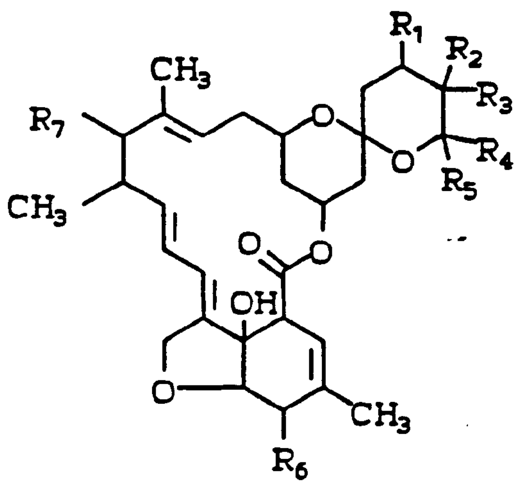

where
$R_1$ is hydrogen, hydroxy, oxo, halogen, =NOH or =$NOCH_3$;
$R_2$ is hydroxy, halogen or cyano; and
$R_3$ is methyl; or
$R_1$ and $R_2$ are combined to form a 23,24-epoxide; and
$R_3$ is methyl; or
$R_1$ and $R_2$ are combined to form a 23,24-double bond; and
$R_3$ is hydroxymethyl, loweralkoxy methyl, loweralkanoyloxymethyl, or halomethyl; and
$R_4$ is an alpha-branched loweralkyl or loweralkenyl group;
$R_5$ is hydrogen or hydroxy;
$R_6$ is hydroxy, loweralkoxy, oxo, =NOH, =$NOCH_3$, triloweralkyl substituted silykoxy, loweralkanoyloxy or phenoxy-loweralkanoyloxy;
$R_7$ is hydrogen, hydroxy, loweralkoxy, loweralkanoyloxy,

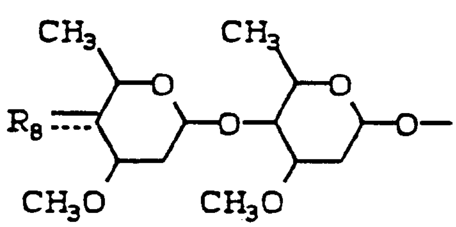

or

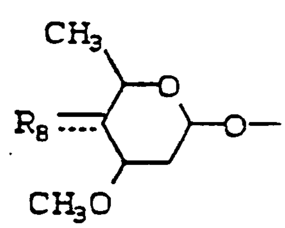

where $R_8$ is connected to the 4′ or 4″ carbon atoms by a single bond and is hydroxy, loweralkanoyloxy, loweralkoxy, amino, N-loweralkylamino, N,N-diloweralkylamino, N-loweralkyl-N-loweralkanoyl amino, N-loweralkyl substituted silyloxy or phenoxyloweralkanoyloxy; or $R_8$ is attached to the 4′ or 4″ carbon atoms by a double bond and is oxo, semicarbazono, N-loweralkylsemicarbazono, N,N-diloweralkylsemicarbazono, loweralkanoylhydrazono, benzoylhydrazono or loweralkylbenzoylhydrazono.

2. A compound of Claim 1 wherein: $R_1$ and $R_2$ together form a 23,24-double bond and $R_3$ is hydroxymethyl, loweralkoxymethyl, loweralkanoyloxymethyl or halomethyl.

3. A compound of Claim 1 wherein: $R_1$ and $R_2$ together form a 23,24-epoxide and $R_3$ is methyl.

4. A compound of Claim 1 wherein:
$R_1$ is hydroxy, oxo, =NOH or =$NOCH_3$;
$R_2$ is hydroxy, cyano or halogen; and
$R_3$ is methyl.

5. The compound of Claim 1, which is 23,24-epoxyavermectin B1a/B1b or 24-cyanoavermectin B2a/B2b.

6. The compound of Claim 1, which is 24-hydroxyavermectin B2a/B2b or 25-hydroxyavermectin B2a/B2b.

7. A process for the preparation of compounds of Claim 1, wherein $R_1$ and $R_2$ are combined to form an epoxide which comprises treating a suitably protected avermectin starting material with oxidizing agent.

8. A process for the preparation of n compound of Claim 1 wherein $R_1$ and $R_2$ are combined to form a 22,23-double bond which comprises treating a suitably protected avermectin starting material with DAST.

9. A method for the treatment of parasitic infections of plants, which comprises treating the infected plant, or the soil in which the injected plant grows with on effective amount of a compound of Claim 1.

10. The use of a compound of Claim 1 for the manufacture of a medicament for the treatment of parasitic infections of animals.

11. A composition useful for the treatment of animals or plants infected with parasites, which comprises an inert carrier and a compound of Claim 1.